(19) Europäisches Patentamt
European Patent Office
Office européen des brevets

(11) Veröffentlichungsnummer: **0 628 310 A1**

(12) # EUROPÄISCHE PATENTANMELDUNG

(21) Anmeldenummer: **94108126.7**

(22) Anmeldetag: **26.05.94**

(51) Int. Cl.[5]: **A61K 31/44**, C07D 405/06, C07D 405/14

(30) Priorität: **08.06.93 DE 4319038**

(43) Veröffentlichungstag der Anmeldung: **14.12.94 Patentblatt 94/50**

(84) Benannte Vertragsstaaten: **AT BE CH DE DK ES FR GB GR IE IT LI LU MC NL PT SE**

(71) Anmelder: **BAYER AG**

**D-51368 Leverkusen (DE)**

(72) Erfinder: **Wild, Hanno, Dr.**
**Am Ausblick 128**
**D-42113 Wuppertal (DE)**
Erfinder: **Bender, Wolfgang, Dr.**
**Kaulbachstrasse 12**
**D-42113 Wuppertal (DE)**
Erfinder: **Häbich, Dieter, Dr.**
**Krummacherstrasse 82**
**D-42115 Wuppertal (DE)**
Erfinder: **Heine, Hans-Georg, Dr.**
**Am Heckerhof 14**
**D-47800 Krefeld (DE)**
Erfinder: **Raddatz, Siegfried, Dr.**
**Jakob-Böhme-Strasse 21**
**D-51065 Köln (DE)**
Erfinder: **Röben, Wolfgang, Dr.**
**Strässchen Siefen 30**
**D-51467 Bergisch Gladbach (DE)**
Erfinder: **Hansen, Jutta, Dr.**
**Pahlkestrasse 98**
**D-42115 Wuppertal (DE)**
Erfinder: **Paessens, Arnold, Dr.**
**Stresemannstrasse 51**
**D-42781 Haan (DE)**

(54) **Verwendung von teilweise bekannten substituierten Chromanen als Arzneimittel, neue Wirkstoffe und Verfahren zu ihrer Herstellung.**

(57) Die vorliegende Erfindung betrifft die Verwendung von substituierten Chromanen der allgemeinen Formel (I)

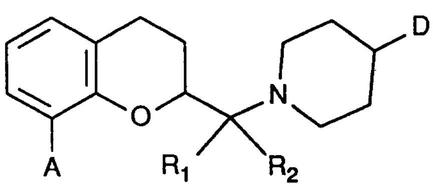

in welcher
die Substituenten die in der Beschreibung angegebene Bedeutung haben zur Herstellung von Arzneimitteln, insbesondere als HIV-Protease inhibierende Mittel, neue Wirkstoffe und Verfahren zu ihrer Herstellung.

EP 0 628 310 A1

Die Erfindung betrifft die Verwendung von teilweise bekannten substituierten Chromanen als Arzneimittel, insbesondere als HIV-Protease inhibierende Mittel, neue Wirkstoffe und Verfahren zu ihrer Herstellung.

Es wurde gefunden, daß die substituierten Chromane der allgemeinen Formel (I)

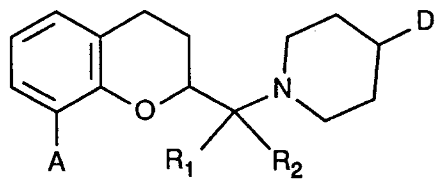

in welcher

A für Wasserstoff, Hydroxy oder geradkettiges oder verzweigtes Alkoxy mit bis zu 8 Kohlenstoffatomen steht,

$R^1$ und $R^2$ für Wasserstoff stehen,

oder

$R^1$ und $R^2$ gemeinsam für die -C = O-Gruppe stehen,

D für einen Rest der Formel $-NR^3-CO-NR^4R^5$ oder

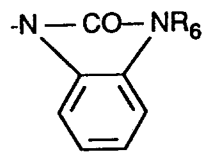

steht,

worin

$R^3$, $R^4$ und $R^6$ gleich oder verschieden sind und Wasserstoff oder geradkettiges oder verzweigtes Alkyl mit bis zu 6 Kohlenstoffatomen bedeuten,

$R^5$ Aryl mit 6 bis 10 Kohlenstoffatomen bedeutet, das gegebenenfalls bis zu 3-fach gleich oder verschieden durch Halogen, Hydroxy, Carboxy, Trifluormethyl, Trifluormethoxy, durch geradkettiges oder verzweigtes Acyl, Alkoxy oder Alkoxycarbonyl mit jeweils bis zu 8 Kohlenstoffatomen oder durch geradkettiges oder verzweigtes Alkyl oder Alkenyl mit jeweils bis zu 8 Kohlenstoffatomen substituiert ist, die ihrerseits durch Hydroxy, Carboxy oder durch geradkettiges oder verzweigtes Alkoxy oder Alkoxycarbonyl mit jeweils bis zu 6 Kohlenstoffatomen substituiert sein können

und deren Racemate, Enantiomere und Salze,

überraschenderweise eine starke inhibierende Wirkung gegen die HIV-Protease besitzen und somit geeignet sind zur Verwendung bei der Bekämpfung von HIV.

Bevorzugt werden Verbindungen der allgemeinen Formel (I) verwendet,

in welcher

A für Wasserstoff, Hydroxy oder geradkettiges oder verzweigtes Alkoxy mit bis zu 6 Kohlenstoffatomen steht,

$R^1$ und $R^2$ für Wasserstoff stehen,

oder

$R^1$ und $R^2$ gemeinsam für die -C = O-Gruppe stehen,

D für einen Rest der Formel $-NR^3-CO-NR^4R^5$ oder

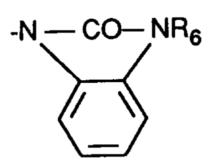

steht,

worin

$R^3$, $R^4$ und $R^6$ gleich oder verschieden sind und Wasserstoff oder geradkettiges oder verzweigtes Alkyl mit bis zu 4 Kohlenstoffatomen bedeuten,

$R^5$ Phenyl oder Naphthyl bedeutet, die gegebenenfalls bis zu 2-fach gleich oder verschieden durch Fluor, Chlor, Brom, Hydroxy, Carboxy, Trifluormethyl, Trifluormethoxy, durch geradkettiges oder verzweigtes Acyl, Alkoxy oder Alkoxycarbonyl mit jeweils bis zu 6 Kohlenstoffatomen, oder durch geradkettiges oder verzweigtes Alkyl oder Alkenyl mit jeweils bis zu 6 Kohlenstoffatomen substituiert sind, die ihrerseits durch Hydroxy, Carboxy oder durch geradkettiges oder verzweigtes Alkoxy oder Alkoxycarbonyl mit jeweils bis zu 4 Kohlenstoffatomen substituiert sein können,

und deren Racemate, Enantiomere und Salze,
bei der Bekämpfung von HIV.

Besonders bevorzugt werden Verbindungen der allgemeinen Formel (I) verwendet,
in welcher

A für Wasserstoff, Hydroxy oder geradkettiges oder verzweigtes Alkoxy mit bis zu 5 Kohlenstoffatomen steht,

$R^1$ und $R^2$ für Wasserstoff stehen,

oder

$R^1$ und $R^2$ gemeinsam für die -C = O-Gruppe stehen,

D für einen Rest der Formel $-NR^3-CO-NR^4R^5$ oder

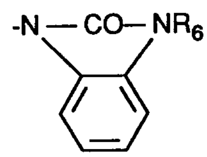

steht,
worin

$R^3$, $R^4$ und $R^6$ gleich oder verschieden sind und Wasserstoff oder geradkettiges oder verzweigtes Alkyl mit bis zu 3 Kohlenstoffatomen bedeuten,

$R^5$ Phenyl oder Naphthyl bedeutet, die gegebenenfalls bis zu 2-fach gleich oder verschieden durch Fluor, Chlor, Brom, Trifluormethyl, Trifluormethoxy, durch geradkettiges oder verzweigtes Acyl, Alkoxy oder Alkoxycarbonyl mit jeweils bis zu 5 Kohlenstoffatomen, oder durch geradkettiges oder verzweigtes Alkyl oder Alkenyl mit jeweils bis zu 5 Kohlenstoffatomen substituiert sind, die ihrerseits durch Hydroxy, Carboxy oder durch geradkettiges oder verzweigtes Alkoxy oder Alkoxycarbonyl mit jeweils bis zu 3 Kohlenstoffatomen substituiert sein können,

und deren Racemate, Enantiomere und Salze,
bei der Bekämpfung von HIV.

Die Erfindung betrifft außerdem neue Verbindungen der allgemeinen Formel (Ia)

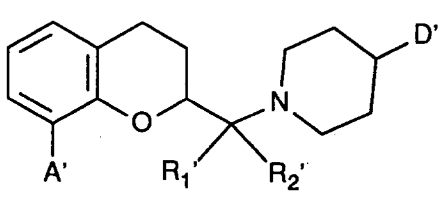

(Ia)

in welcher

A' für Wasserstoff, Hydroxy oder geradkettiges oder verzweigtes Alkoxy mit bis zu 8 Kohlenstoffatomen bedeutet,

$R^{1'}$ und $R^{2'}$ für Wasserstoff stehen,

oder

$R^{1'}$ und $R^{2'}$ gemeinsam für die -C = O-Gruppe stehen,

D für einen Rest der Formel $-NR^{3'}-CO-NR^{4'}R^{5'}$ oder

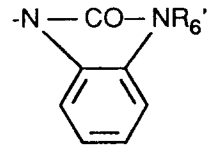

steht,
worin

| | |
|---|---|
| $R^{3'}$, $R^{4'}$ und $R^{6'}$ | gleich oder verschieden sind und Wasserstoff oder geradkettiges oder verzweigtes Alkyl mit bis zu 6 Kohlenstoffatomen bedeuten, |
| $R^{5'}$ | Aryl mit 6 bis 10 Kohlenstoffatomen bedeutet, das gegebenenfalls bis zu 3-fach gleich oder verschieden durch Halogen, Hydroxy, Carboxy, Trifluormethyl, Trifluormethoxy, durch geradkettiges oder verzweigtes Acyl, Alkoxy oder Alkoxycarbonyl mit jeweils bis zu 8 Kohlenstoffatomen oder durch geradkettiges oder verzweigtes Alkyl oder Alkenyl mit jeweils bis zu 8 Kohlenstoffatomen substituiert ist, die ihrerseits durch Hydroxy, Carboxy oder durch geradkettiges oder verzweigtes Alkoxy oder Alkoxycarbonyl mit jeweils bis zu 6 Kohlenstoffatomen substituiert sein können |

und deren Racemate, Enantiomere und Salze,
mit der Maßgabe, daß D' nicht für den Rest der Formel

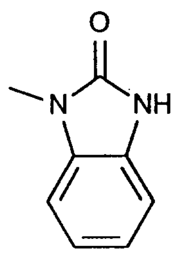

stehen darf, wenn im Fall des Racemats und der Enantiomere A', $R^{1'}$ und $R^{2'}$ Wasserstoff bedeuten, oder im Fall des Racemats, A' für Methoxy steht und $R^1$ und $R^2$ Wasserstoff bedeuten.

Physiologisch unbedenkliche Salze der substituierten Chromane der allgemeinen Formeln (I) und (Ia) können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin.

Die erfindungsgemäßen Verbindungen der allgemeinen Formeln (I) und (Ia) können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Außerdem wurden Verfahren zur Herstellung der erfindungsgemäßen Verbindungen der allgemeinen Formeln (I) und (Ia) gefunden, dadurch gekennzeichnet, daß man
im Fall, daß D und D' jeweils entsprechend für die Reste der Formeln $-NR^3-CO-NR^4R^5$ oder $-NR^{3'}-CO-NR^{4'}R^{5'}$, stehen,

[A] Verbindungen der allgemeinen Formel (II)

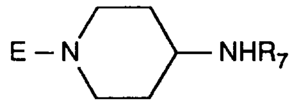 (II)

in welcher

$R^7$ die oben angegebene Bedeutung von $R^3$ und $R^{3'}$ umfaßt

und

E für eine Aminoschutzgruppe, vorzugsweise für Benzyl steht,

zunächst durch Umsetzung mit Isocyanaten der allgemeinen Formel (III)

$O=C=N\text{-}R^8$ (III)

in welcher

$R^8$ die oben angegebene Bedeutung von $R^5$ und $R^{5'}$ umfaßt,

in inerten Lösemitteln, gegebenenfalls in Anwesenheit einer Base und/oder eines Hilfsmittels, in die Verbindungen der allgemeinen Formel (IV)

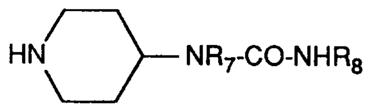 (IV)

in welcher

$R^7$ und $R^8$ die oben angegebene Bedeutung haben,

überführt,

und anschließend mit Verbindungen der allgemeinen Formel (V)

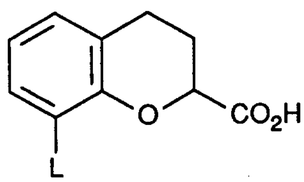 (V)

in welcher

L die oben angegebene Bedeutung von A und A' umfaßt, aber nicht für Hydroxy steht,

ebenfalls in inerten Lösemitteln, gegebenenfalls in Anwesenheit einer Base und eines Hilfsstoffes umsetzt,

oder

[B] im Fall, daß D und D' jeweils entsprechend für die Reste der Formeln

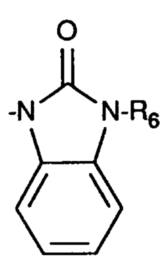 oder 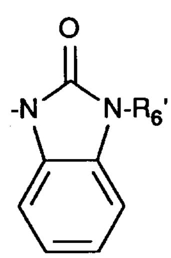

stehen,

Verbindungen der allgemeinen Formel (V) mit Verbindungen der allgemeinen Formel (VI)

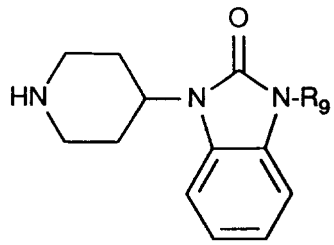

(VI)

in welcher

$R^9$ die oben angegebene Bedeutung von $R^6$ und $R^{6'}$ umfaßt,

in inerten Lösemitteln, in Anwesenheit einer Base und/oder Hilfsmittels umsetzt,
und im Fall, $R^1/R^{1'},R^2$ = H die Carbonylfunktion nach üblichen Methoden reduziert,
und im Fall L = OH die entsprechende Methoxyverbindung mit Säuren spaltet,
und im Fall L = Alkoxy die entsprechenden Hydroxyverbindungen verethert,
und im Fall $R^7,R^4/R^{4'}$ und/oder $R^9 \neq$ H gegebenenfalls eine Alkylierung durchführt.

Die erfindungsgemäßen Verfahren können durch folgendes Formelschema beispielhaft erläutert werden:

[A]

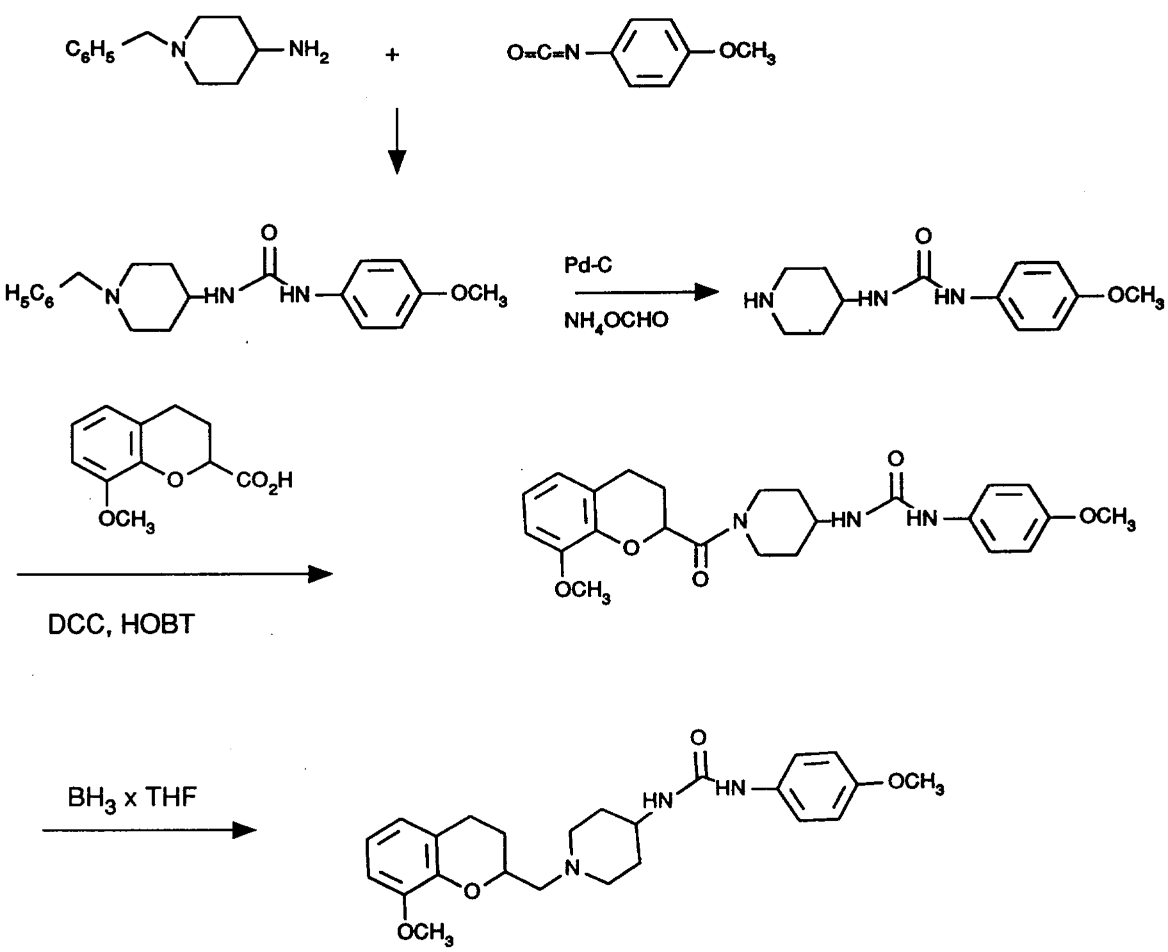

[B]

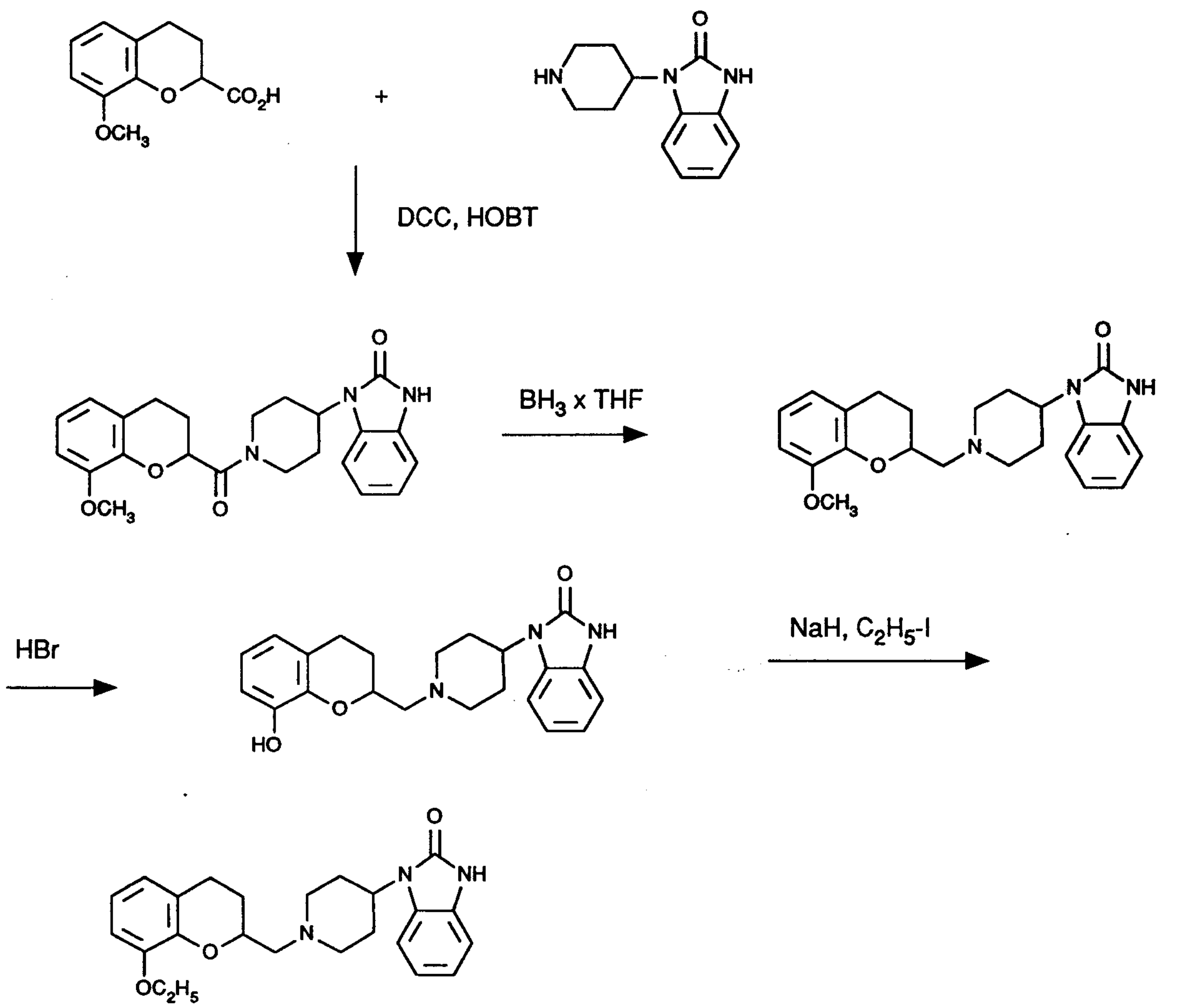


Aminoschutzgruppen im Rahmen der Erfindung sind die üblichen in der Peptid-Chemie verwendeten Aminoschutzgruppen. Hierzu gehören bevorzugt:
Benzyloxycarbonyl, tert.Butoxycarbonyl oder Allyloxycarbonyl.

Als Lösemittel eignen sich die üblichen inerten Lösemittel, die sich unter den Reaktionsbedingungen nicht verändern. Hierzu gehören bevorzugt organische Lösemittel wie Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester, Pryridin, Triethylamin oder Picolin. Ebenso ist es möglich, Gemische der genannten Lösemittel zu verwenden. Besonders bevorzugt sind Dichlormethan, Chloroform, Dimethylformamid oder Tetrahydrofuran.

Als Basen für die erfindungsgemäßen Verfahren können im allgemeinen anorganische oder organische Basen eingesetzt werden. Hierzu gehören vorzugsweise Alkalihydroxide wie zum Beispiel Lithiumhydroxid, Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate, wie Calcium- oder Caesiumcarbonat, oder Alkali- oder Erdalkalialkoholate oder -amide wie Natrium- oder Kaliummethanolat, Natrium- oder Kaliumethanolat oder Kalium-tert.butylat, oder Lithiumdiisopropylamid (LDA), oder organische Amine (Trialkyl($C_1$-$C_6$)-amine) wie Triethylamin, oder Heterocyclen wie 1,4-Diazabicyclo[2.2.2]octan (DABCO), 1,8-Diazabicyclo-[5.4.0]undec-7-en (DBU), Pyridin, Diaminopyridin, N-Methylpiperidin oder Morpholin. Es ist auch möglich als Basen Alkalimetalle, wie Natrium oder deren Hydride wie Natriumhydrid einzusetzen. Bevorzugt ist Triethylamin oder N-Methylmorpholin.

Im allgemeinen setzt man die Base in einer Menge von 0,05 mol bis 10 mol, bevorzugt von 1 mol bis 2 mol, jeweils bezogen auf 1 mol der Verbindungen der Formeln (III), (V) und (VI) ein.

Die erfindungsgemäßen Verfahren werden im allgemeinen in einem Temperaturbereich von -100 °C bis + 100 °C, bevorzugt von 0 °C bis 80 °C, durchgeführt.

Die erfindungsgemäßen Verfahren werden im allgemeinen bei Normaldruck durchgeführt. Es ist aber auch möglich, die Verfahren bei Überdruck oder bei Unterdruck durchzuführen (z.B. in einem Bereich von 0,5 bis 5 bar).

Als Hilfsstoffe werden bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N'-Diethyl-, N,N'-Diisopropyl-, N,N'-Dicyclohexylcarbodiimid, N-(3-Dimethyl-aminoisopropyl)-N'-ethyl-carbodiimid-Hydrochlorid, N-Cyclohexyl-N'-(2-morpholinoethyl)-carbodiimid-metho-p-toluolsulfonat, oder Carbonylverbindungen wie Carbonyldiimidazol, oder 1,2-Oxazoliumverbindungen wie 2-Ethyl-5-phenyl-1,2-oxazolium-3-sulfat oder 2-tert-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)phosphonium-hexafluorophosphat oder 1-Hydroxybenzotriazol.

Außerdem können beispielsweise Alkalicarbonate, z.B. Natrium- oder Kaliumcarbonat oder -hydrogencarbonat, oder organische Basen wie Trialkylamine, z.B. Triethylamin, Ethyldiisopropylamin, N-Ethylmorpholin, N-Methylpiperidin oder N-Methylmorpholin eingesetzt werden. Bevorzugt ist N-Methylmorpholin.

Die Hilfsstoffe werden in einer Menge von 1,0 Mol bis 3,0 Mol, bevorzugt 1,0 bis 1,2 Mol, bezogen auf jeweils 1 Mol der Verbindungen der allgemeinen Formeln (III), (IV) und (VI) eingesetzt.

Die Reaktionen werden in einem Temperaturbereich von -30 °C bis 100 °C, vorzugsweise bei 0 °C bis 30 °C und bei Normaldruck, durchgeführt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0 °C bis +100 °C, bevorzugt von +20 °C bis +80 °C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol, bezogen auf 1 Mol des Esters, eingesetzt. Besonders bevorzugt verwendet man molare Mengen der Reaktanden.

Die Verseifung von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan, Tetrahydrofuran oder Dichlormethan.

Die Abspaltung der Aminoschutzgruppen kann nach üblicher Methode mit Säuren, wie beispielsweise Chlorwasserstoffsäure oder Trifluoressigsäure erfolgen.

Die Abspaltung der Aminoschutzgruppen erfolgt in an sich bekannter Weise unter sauren oder basischen Bedingungen, oder reduktiv durch katalytische oder Transfer-Hydrierung beispielsweise mit Pd/C in organischen Lösemitteln wie Ethern, z.B. Tetrahydrofuran oder Dioxan, oder Alkoholen z.B., Methanol, Ethanol oder Isopropanol.

Die Hydrierung erfolgt im allgemeinen in einem Temperaturbereich von 0 °C bis 80 °C, vorzugsweise von 0 °C bis 40 °C.

Im allgemeinen wird die Hydrierung bei erhöhtem Druck von 2 bar bis 8 bar, vorzugsweise von 3 bis 5 bar, durchgeführt.

Die Abspaltung der Benzylschutzgruppen erfolgt vorzugsweise mit Ammoniumformiat/Palladium/C in Ethanol/Wasser in einem Temperaturbereich von 20 °C bis 80 °C, vorzugsweise bei 50 °C und Normaldruck.

Die Alkylierung von Aminogruppen erfolgt entweder mit Sulfonsäureestern oder substituierten oder unsubstituierten ($C_1$-$C_8$)-Dialkyl- oder ($C_1$-$C_8$)-Diarylsulfonate, vorzugsweise Methyliodid oder Dimethylsulfat, oder mit Formaldehyd/Natriumborhydrid in einem der oben aufgeführten Ether, vorzugsweise Tetrahydrofuran, in Anwesenheit von Säuren.

Die Alkylierung erfolgt im allgemeinen in einem der oben aufgeführten Lösemitteln, vorzugsweise in Dimethylformamid in einem Temperaturbereich von 0 °C bis +70 °C, vorzugsweise von 0 °C bis +30 °C und Normaldruck.

Die Reduktion der Carbonylfunkionen erfolgt im allgemeinen mit Reduktionsmitteln, wie beispielsweise Lithiumaluminiumhydrid, mit Boranlösung in Tetrahydrofuran oder mit Boran-Dimethylsulfid (Komplex in

Tetrahydrofuran) in einem Temperaturbereich von 0 °C bis +70 °C, bevorzugt von +20 °C bis +65 °C und Normaldruck und anschließender Aufnahme in Säuren. Bevorzugt ist Boran-Lösung in Tetrahydrofuran.

Als Säuren für einzelne Verfahrensschritte eignen sich im allgemeinen Protonensäuren wie beispielsweise Salzsäure oder Schwefelsäure. Bevorzugt wird Schwefelsäure eingesetzt.

Die Säure wird im allgemeinen in einer Menge von 1 mol bis 20 mol, bevorzugt von 1 mol bis 5 mol, jeweils bezogen auf 1 mol des Reaktanden, eingesetzt.

Die Veretherung der erfindungsgemäßen Verbindungen (A/A' (L) ≠ OH) erfolgt mit einem der oben aufgeführten Alkylierungsmittel in Anwesenheit einer der oben aufgeführten Lösemittel und Basen, vorzugsweise mit Kalium-tert.butylat und Natriumhydrid.

Die Umsetzungen werden im allgemeinen in einem Temperaturbereich von -20 °C bis +80 °C, bevorzugt von 0 °C bis +60 °C durchgeführt.

Im allgemeinen wird die Umsetzung bei Normaldruck durchgeführt. Es ist aber auch möglich, bei Unterdruck oder bei Überdruck zu arbeiten (z.B. von 0,5 bis 5 bar).

Die Freisetzung der Hydroxyfunktion (A/A' (L) = OH) erfolgt mit Wasserstoffsäuren, vorzugsweise Bromwasserstoffsäure in der Siedehitze und Normaldruck.

Die Verbindungen der allgemeinen Formeln (IV) und (VI) sind teilweise bekannt oder neu und können dann beispielsweise wie oben beschrieben hergestellt werden.

Die Verbindungen der allgemeinen Formeln (II), (III) und (V) sind an sich bekannt.

Die hier beschriebenen Inhibitoren der allgemeinen Formel (I) und die neuen Verbindungen der allgemeinen Formel (Ia) sind Inhibitoren der HIV-Protease und sind als solche für alle Zwecke einsetzbar, für die Enzyminhibitoren geeignet sind. Dies ist zum Beispiel der Einsatz in der Diagnostik, um die Präzision und Selektivität von Enzymaktivitätsmessungen zu verbessern. Bei der Affinitätschromatographie können sie als Affinitätslabel dienen und in der Forschung kann man sie zur Aufklärung von Reaktionsmechanismen und der Spezifität enzymatischer Reaktionen verwenden.

Darüber hinaus wurde überraschend gefunden, daß die Verbindungen der allgemeinen Formeln (I) und (Ia) eine Wirkung gegen Retroviren besitzen. Dies wird mit einem HIV-spezifischen Protease-Enzymtest belegt.

Die Ergebnisse der unten aufgeführten Beispiele wurden nach dem in den folgenden Literaturangaben [vgl. Hansen, J., Billich, S., Schulze, T., Sukrow, S. and Mölling, K. (1988), EMBO Journal, Vol, 7, No. 6, pp. 1785 - 1791] beschriebenen HIV-Testsystem ermittelt: Gereinigte HIV-Protease wurde mit synthetischem Peptid, das eine Schnittstelle im Gag-Precursor-Protein imitiert und eine in vivo-Spaltstelle der HIV-Protease darstellt, inkubiert. Die entstandenen Spaltprodukte des synthetischen Peptids wurden über Reverse Phase High Performance Liquid Chromatography (RP-HPLC) analysiert. Die angegebenen $IC_{50}$-Werte beziehen sich auf die Substanzkonzentration, die unter den oben aufgeführten Testbedingungen eine 50%ige Hemmung der Protease-Aktivität bewirkt.

Tabelle 1

| Bsp.-Nr. | % Inhibition bei 200 µg/ml | $IC_{50}$ (µM) |
|---|---|---|
| 1 | 99 | |
| 21 | 65 | |
| 24 | 99 | |
| 25 | 74 | |
| 27 | 81 | |
| 28 | 94 | |
| 30 | 99 | 0,0002 |
| 33 | 99 | |

HIV-Infektion in Zellkultur

Der HIV-Test wurde mit geringen Modifikationen nach der Methode von Pauwels et al. [vgl. Journal of Virological Methods 20, (1988), 309-321] durchgeführt.

Normale menschliche Blutlymphozyten (PBL's) wurden über Ficoll-Hypaque angereichert und im RPMI 1640, 20% fötales Kälberserum mit Phythaemagglutinin (90 µg/ml) und Interleukin-2 (40 U/ml) stimuliert. Zur Infektion mit dem infektiösen HIV wurden PBL's pelletiert und das Zellpellet wurde anschließend in 1 ml

HIV-Virusadsorptionslösung suspendiert und 1 Stunde bei 37 °C inkubiert.

Alternativ wurden HIV-suszeptible H9-Zellen anstelle von normalen menschlichen Blutlymphozyten zur Testung der antiviralen Effekte der erfindungsgemäßen Verbindungen eingesetzt.

Die Virusadsorptionslösung wurde zentrifugiert und das infizierte Zellpellet in Wachstumsmedium aufgenommen, so daß 1 x $10^5$ Zellen pro ml eingestellt waren. Die derart infizierten Zellen wurden zu 1 x $10^4$ Zellen/Napf in die Näpfe von 96er Mikrotiterplatten pipettiert.

Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen in unterschiedlichen Konzentrationen, ausgehend von den Näpfen der 3. vertikalen Reihe der Mikrotiterplatte, von der die Prüfsubstanzen in 2er Schritten $2^{10}$fach verdünnt wurden.

Die Testansätze wurden so lange bei 37 °C inkubiert, bis in der unbehandelten Viruskontrolle die für das HIV typische Syncytienbildung auftrat (zwischen Tag 3 und 6 nach Infektion), die dann mikroskopisch ausgewertet wurde. In der unbehandelten Viruskontrolle resultierten unter diesen Testbedingungen etwa 20 - 50 Syncytien, währen die unbehandelte Zellkontrolle keine Syncytien aufwies.

Die $IC_{50}$-Werte wurden als die Konzentration der behandelten und infizierten Zellen ermittelt, bei der 50% (ca. 10 - 20 Syncytien) der virusinduzierten Syncytien durch die Behandlung mit der erfindungsgemäßen Verbindung unterdrückt waren.

Es wurde nun gefunden, daß die erfindungsgemäßen Verbindungen HIV infizierte Zellen vor der virusinduzierten Zellzerstörung schützen.

Tabelle 2

| Bsp.-Nr. | $IC_{50}$ (μM, PBL-Zellkultur) |
|---|---|
| 30 | 10 |
| 31 | 10 |

Die erfindungsgemäßen Verbindungen der allgemeinen Formeln (I) und (Ia) stellen wertvolle Wirkstoffe zur Behandlung und Prophylaxe von Erkrankungen, hervorgerufen durch Retroviren, in der Human- und Tiermedizin dar.

Als Indikationsgebiete in der Humanmedizin können beispielsweise genannt werden:

1.) Die Behandlung und Prophylaxe von menschlichen Retrovirusinfektionen.

2.) Für die Behandlung oder Prophylaxe von durch HIV I (Virus der humanen Immundefizienz; früher HTLV III/LAV genannt) und HIV II verursachten Erkrankungen (AIDS) und den damit assoziierten Stadien wie ARC (AIDS related complex) und LAS (Lymphadenopathie-Syndrom) sowie der durch dieses Virus verursachten Immunschwäche und Encephalopathie.

3.) Für die Behandlung oder die Prophylaxe einer HTLV-I oder HTLV-II Infektion.

4.) Für die Behandlung oder die Prophylaxe des AIDS-carrier Zustandes (AIDS-Überträger-Zustand).

Als Indikationen in der Tiermedizin können beispielsweise angeführt werden:

Infektionen mit

a) Maedi-visna (bei Schafen und Ziegen)

b) progressivem Pneumonievirus (PPV) (bei Schafen und Ziegen)

c) caprine arthritis encephalitis Virus (bei Schafen und Ziegen)

d) Zwoegersiekte Virus (bei Schafen)

e) infektiösem Virus der Anämie (des Pferdes)

f) Infektionen verursacht durch das Katzenleukämievirus

g) Infektionen verursacht durch das Virus der Katzen-Immundefizienz (FIV)

h) Infektionen verursacht durch das Virus der Affen-Immundefizienz (SIV)

Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formeln (I) und (Ia) enthalten oder die aus einem oder mehreren Wirkstoffen der Formeln (I) und (Ia) bestehen, sowie Verfahren zur Herstellung dieser Zubereitungen.

Die Wirkstoffe der Formeln (I) und (Ia) sollen in den oben aufgeführten pharmazeutischen Zubereitungen, in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den Verbindungen der Formeln (I) und (Ia) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Ausgangsverbindungen

Beispiel I

N-(1-Benzyl-4-piperidyl)-N'-phenylharnstoff

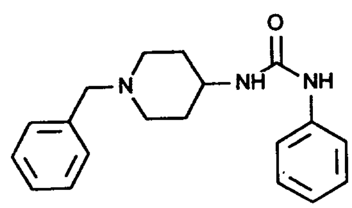

Zu einer Lösung von 1,90 g (10,0 mmol) 4-Amino-1-benzylpiperidin und 1,46 ml (10,5 mmol) Triethylamin in 40 ml Dichlormethan werden langsam 1,14 ml (10,5 mmol) Phenylisocyanat getropft. Die Reaktionsmischung darf 1 h bei Raumtemperatur nachrühren, dann wird im Vakuum zur Trockne eingedampft. Der Rückstand wird mit 50 ml Diethylether versetzt, gut durchgerührt, durch Filtration abgetrennt, mit 5 ml Diethylether gewaschen und im Hochvakuum getrocknet. Man erhält 2,62 g (85%) der Titelverbindung als farblose Kristalle.

Schmp.: 161-162,5 °C

$R_f$ = 0,24 (Dichlormethan/Methanol (9:1))

MS (FAB) m/z: 310 $(M+H)^+$

Beispiel II

N-(4-Piperidyl)-N'-phenylharnstoff

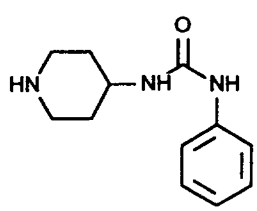

Eine Suspension von 2,48 g (8,00 mmol) der Verbindung aus Beispiel I in einem Gemisch aus 38 ml Ethanol und 3,8 ml Wasser wird mit 1,26 g (20,0 mmol) Ammoniumformiat und dann mit 1,26 g Palladium auf Kohle versetzt und 1 h auf 50 °C erwärmt. Danach darf die Reaktionsmischung abkühlen. Der feste

Anteil wird durch Filtration abgetrennt und mit 5 ml Ethanol gewaschen. Das Filtrat wird im Vakuum zur Trockne eingeengt, der Rückstand wird mit Diethylether verrieben, abgesaugt, mit 5 ml Diethylether gewaschen und im Hochvakuum getrocknet. Man erhält 1,53 g (87%) der Titelverbindung als farblose Kristalle.

Schmp.: 160-161 °C

$R_f$ = 0,01 (Dichlormethan/Methanol (8:2)).

MS (FAB) m/z: 220 $(M+H)^+$

Wie für Beispiel I beschrieben, erhält man durch Umsetzung von 4-Amino-1-benzylpiperidin mit verschiedenen Isocyanaten die in Tabelle I aufgeführten Produkte:

Tabelle I:

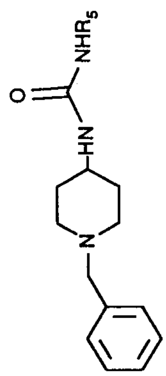

| Bsp.-Nr. | $R^5$ | Ausbeute (%) | MS (FAB) m/z $(M+H)^+$ | $R_f$/Laufmittelverhältnis b) | Schmp. (°C) |
|---|---|---|---|---|---|
| III | $4-CH_3-C_6H_4-$ | 98 | 323 a) | 0,27, I (9:1) | 199 |
| IV | $3-CF_3-C_6H_4-$ | 87 | 377 a) | 0,30, I (9:1) | 179 |
| V | $4-CF_3-C_6H_4-$ | 64 | 377 a) | 0,25, I (9:1) | 158 |
| VI | $2\text{--}F, 4-F-C_6H_4-$ | 70 | 345 a) | 0,26, I (9:1) | 159 |
| VII | $4-H_5C_2OOC-C_6H_4-$ | 91 | 382 | 0,32, I (9:1) | 125 |
| VIII | 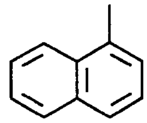 | 97 | 360 | 0,31, I (9:1) | 194 |
| IX | $4-CH_3CO-C_6H_4$ | 95 | 352 | 0,19, I (9:1) | 138 |
| X | $3-CH_3OOC-, 5-CH_3OOC-C_6H_4-$ | 92 | 426 | 0,26, I (9:1) | 106 |
| XI | $4-H_5C_2OOC-CH=CH-C_6H_4-$ | 95 | 408 | 0,60, I (9:1) | 177 |

a) MS (EI, 70 ev) m/z $(M)^+$ b) I = Dichlormethan/Methanol

Wie für Beispiel II beschrieben erhält man aus den entsprechenden Startmaterialien die in Tabelle II aufgeführten Produkte:

Tabelle II:

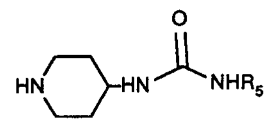

| Bsp.-Nr. | $R^5$ | Ausbeute (%) | MS (FAB) m/z $(M+H)^+$ | Schmp. (°C) | Startmaterial aus Beispiel |
|---|---|---|---|---|---|
| XII | $4-CH_3-C_6H_4-$ | 70 | 234 | 178 | III |
| XIII | $3-CF_3-C_6H_4-$ | 80 | 288 | 139 | IV |
| XIV | $4-CF_3-C_6H_4-$ | 83 | 288 | 187 | V |
| XV | $2-F-, 4-F-C_6H_4-$ | 76 | 256 | 172 | VI |
| XVI | $4-H_5C_2OOC-C_6H_4-$ | 86 | 292 | 173 | VII |
| XVII | 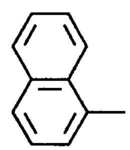 | 65 | 270 | 191 | VIII |
| XVIII | $4-CH_3CO-C_6H_4$ | 68 | 262 | amorphes Pulver | IX |
| XIX | $3-CH_3OOC-, 5-CH_3OOC-C_6H_4-$ | 61 | 336 | 240 | X |
| XX | $4-H_5C_2OOC-CH=CH-C_6H_4-$ [a)] | 66 | 320 | 123 | XI |

a) Bei der Abspaltung der Benzylgruppe erfolgt gleichzeitig die Sättigung der Doppelbindung

Herstellungsbeispiele

Beispiel 1

8-Methoxy-2-[4-(phenylaminocarbonyl-amino)piperidylcarbonyl]chroman

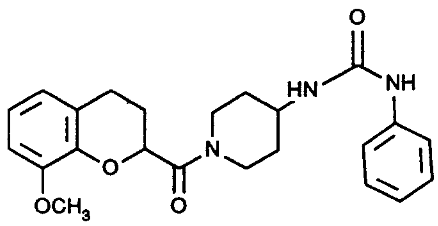

Eine auf 0 °C gekühlte Lösung von 770 mg (3,51 mmol) 8-Methoxy-chromancarbonsäure und 586 mg (3,83 mmol) 1-Hydroxybenzotriazol in 20 ml wasserfreiem Dichlormethan wird mit 723 mg (3,51 mmol) Dicyclohexylcarbodiimid versetzt und 5 min gerührt. Danach wird eine Lösung von 700 mg (3,19 mmol) der Verbindung aus Beispiel II und 0,88 ml (8,00 mmol) N-Methylmorpholin in 20 ml Dichlormethan zugetropft, und die Reaktionsmischung darf 1 h bei Raumtemperatur rühren. Dann trennt man den entstandenen Harnstoff durch Filtration ab, engt das Filtrat im Vakuum ein und reinigt das Rohprodukt durch Chromatographie an 150 g Kieselgel (Dichlormethan/Methanol 95:5). Die produkthaltigen Fraktionen werden gesammelt, das Lösemittel wird im Vakuum abgedampft, und der Rückstand mit Diethylether verrieben, abgesaugt und im Hochvakuum getrocknet Man erhält 1,24 g (95%) der Titelverbindung als farblose Kristalle.
Schmp.: 215-216 °C
$R_f$ = 0,17 (Dichlormethan/Methanol (95:5))
MS (FAB) m/z = 410 $(M+H)^+$

Beispiel 2

8-Methoxy-2-[4-(phenylaminocarbonyl-amino)piperidylmethyl]chroman

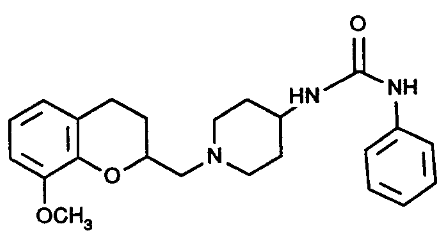

Zu einer gerührten Lösung von 819 mg (2,00 mmol) der Verbindung aus Beispiel 1 in 25 ml wasserfreiem Tetrahydrofuran werden 10,0 ml (10,00 mmol) einer 1 M Lösung von Boran in THF zugetropft. Die Reaktionsmischung wird 1 h bei Raumtemperatur gerührt, in 40 ml 5 N HCl eingetragen und 15 min auf 90 °C erwärmt. Nach dem Abkühlen wird mit konzentrierter Natronlauge pH 11 eingestellt und zweimal mit 20 ml Ethylacetat extrahiert. Die vereinigten organischen Extrakte werden über $MgSO_4$ getrocknet, im Vakuum eingeengt und durch Chromatographie an 25 g Kieselgel (Dichlormethan/Methanol 9:1) gereinigt. Man erhält 659 mg (83%) der Titelverbindung als farblose Kristalle.
Schmp.: 163 °C
$R_f$ = 0,29 (Dichlormethan/Methanol (9:1))
MS (FAB) m/z = 396 $(M+H)^+$

Wie für Beispiel 1 beschrieben erhält man durch Kondensation von 8-Methoxychromancarbonsäure mit den entsprechenden Startmaterialien die in Tabelle 1 aufgeführten Produkte:

Tabelle 1:

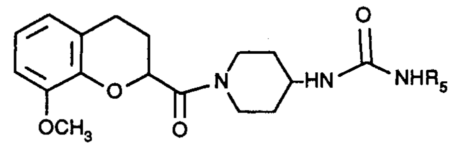

| Bsp.-Nr. | $R^5$ | Ausbeute (%) | MS (FAB) m/z $(M+H)^+$ | $R_f$/Laufmittel-* verhältnis | Schmp. (°C) | Startmaterial aus Beispiel |
|---|---|---|---|---|---|---|
| 3 | $4-CH_3-C_6H_4-$ | 41 | 424 | 0,49, I (95:5) | 196 | XII |
| 4 | $3-CF_3-C_6H_4-$ | 73 | 478 | 0,52, I (9:1) | 203 | XIII |
| 5 | $4-CF_3-C_6H_4-$ | 76 | 478 | 0,43, I (9:1) | 113 | XIV |
| 6 | 2-F-, $4-F-C_6H_4-$ | 95 | 446 | 0,57, I (9:1) | 200 | XV |
| 7 | $4-H_5C_2OOC-C_6H_4-$ | 29 | 482 | 0,33, I (9:1) | 143 | XVI |
| 8 | 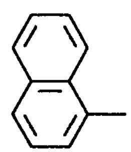 | 31 | 460 | 0,43, I (9:1) | 218 | XVII |
| 9 | $4-CH_3CO-C_6H_4$ | 64 | 452 | 0,44, I (9:1) | 178 | XVIII |
| 10 | $3-CH_3OOC-$, $5-CH_3OOC-C_6H_4-$ | 76 | 526 | 0,41, I (9:1) | 186 | XIX |
| 11 | $4-H_5C_2OOC-CH=CH-C_6H_4-$ | 37 | 510 | 0,53, I (9:1) | 135 | XX |

*) I = Dichlormethan / Methanol

Wie für Beispiel 2 beschrieben erhält man durch Reduktion der entsprechenden Amide (Startmaterialien) die in Tabelle 2 aufgeführten Produkte:

Tabelle 2:

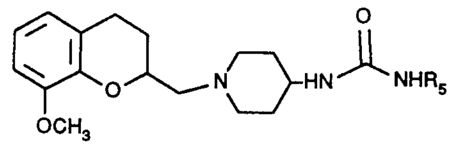

| Bsp.-Nr. | $R^5$ | Ausbeute (%) | MS (FAB) m/z $(M+H)^+$ | $R_f$/Laufmittel-[b] verhältnis | Schmp. (°C) | Startmaterial aus Beispiel |
|---|---|---|---|---|---|---|
| 12 | $4\text{-}CH_3\text{-}C_6H_4\text{-}$ | 59 | 410 | 0,35, I (9:1) | 169 | 3 |
| 13 | $3\text{-}CF_3\text{-}C_6H_4\text{-}$ | 68 | 464 | 0,40, I (9:1) | 168 | 4 |
| 14 | $4\text{-}CF_3\text{-}C_6H_4\text{-}$ | 77 | 464 | 0,37, I (9:1) | 169 | 5 |
| 15 | $2\text{-}F\text{-}, 4\text{-}F\text{-}C_6H_4\text{-}$ | 57 | 431 | 0,35, I (9:1) | 183 | 6 |
| 16 | $4\text{-}H_5C_2OOC\text{-}C_6H_4\text{-}$ | 59 | 468 | 0,39, I (9:1) | 178 | 7 |
| 17 | 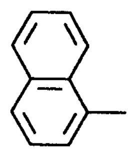 | 67 | 446 | 0,24, I (9:1) | 185 | 8 |
| 18 | $4\text{-}CH_3\text{-}CH_2\text{-}C_6H_4$ [a] | 40 | 424 | 0,13, I (9:1) | 171 | 9 |
| 19 | $3\text{-}CH_3OOC, 5\text{-}CH_3OOC\text{-}C_6H_4\text{-}$ | 41 | 512 | 0,41, I (9:1) | 179 | 10 |
| 20 | $4\text{-}HO\text{-}(CH_2)_3\text{-}C_6H_4\text{-}$ [a] | 24 | 454 | 0,24, I (9:1) | Öl | 11 |

a) zusätzlich zum Amid reduziert b) Dichlormethan/Methanol

Wie für Beispiel 1 beschrieben erhält man durch Kondensation der entsprechenden Chromancarbonsäuren mit 4-(2-Oxo-1-benzimidazolinyl)piperidin die in Tabelle 3 aufgeführten Produkte:

Tabelle 3:

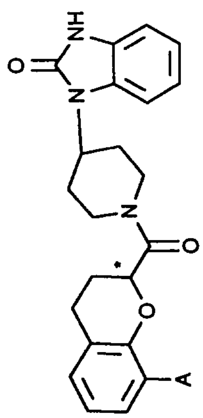

| Bsp.-Nr. | A | * | MS (FAB) m/z $(M+H)^+$ | $R_f$ /Laufmittel a) | Ausbeute |
|---|---|---|---|---|---|
| 21 | H | Racemat | 378 | 0,47, II | 87% |
| 22 | H | (S)-Enantiomer | 378 | 0,47, II | 83% |
| 23 | H | (R)-Enantiomer | 378 | 0,47, II | 79% |
| 24 | $OCH_3$ | Racemat | 408 | 0,42, II | 85% |
| 25 | $OCH_3$ | (S)-Enantiomer | 408 | 0,42, II | 84% |
| 26 | $OCH_3$ | (R)-Enantiomer | 409 | 0,42, II | 90% |

a) II = Essigester/Aceton 5:3

Wie für Beispiel 2 beschrieben erhält man durch Reduktion der Amide die in Tabelle 4 aufgeführten Produkte:

Tabelle 4:

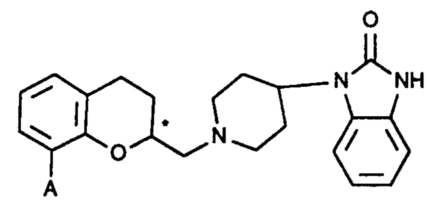

| Bsp.-Nr. | A | * | MS (FAB) m/z $(M+H)^+$ | $R_f$ /Laufmittel [a)] | $[\alpha]_D$ | Ausbeute | Startmaterial aus Beispiel |
|---|---|---|---|---|---|---|---|
| 27 | H | Racemat | 364 | 0,26, II | - | 77% | 21 |
| 28 | H | (S)-Enantiomer EE > 99% | 364 | 0,26, II | +53,9 (c=0,6 THF) | 74% | 22 |
| 29 | H | (R)-Enantiomer EE > 99% | 364 | 0,26, II | -51,2 (c=0,6, THF) | 83% | 23 |
| 30 | $OCH_3$ | Racemat | 394 | 0,21, II | - | 75% | 24 |
| 31 | $OCH_3$ | (S)-Enantiomer EE > 99% | 394 | 0,21, II | +47,0° (c = 1, $CHCl_3$) | 89% | 25 |
| 32 | $OCH_3$ | (R)-Enantiomer EE > 99% | 394 | 0,21, II | -50,5° (c = 1, $CHCl_3$) | 85% | 26 |

a) II = Essigester/Aceton 5:3

Beispiel 33

8-Hydroxy-2-[4-(2-oxo-1-benzimidazolyl)piperidylmethyl]chroman

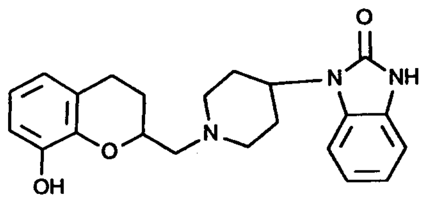

1 g (2,54 mmol) des Methylethers aus Beispiel 30 wird in 20 ml Bromwasserstoffsäure 3 h unter Rückfluß erhitzt. Dann wird mit halbkonz. Natronlauge alkalisch gestellt, 3 mal mit Essigester extrahiert, und die vereinigten organischen Phasen werden getrocknet ($MgSO_4$) und eingeengt. Der Rückstand wird an Kieselgel mit Dichlormethan / Methanol (40:1) als Eluens chromatographiert.
Ausbeute: 97%
$R_f$ = 0,31 (Dichlormethan/Methanol 10:1)
MS (EI): m/z = 379 $(M+H)^+$

Beispiel 34

8-Ethoxy-2-[4-(2-oxo-1-benzimidazolyl)piperidylmethyl]chroman

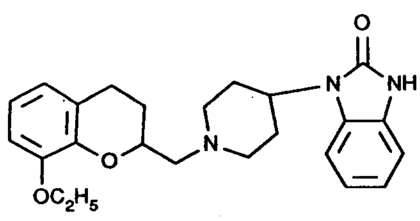

50 mg (0,13 mmol) der Verbindung aus Beispiel 33 werden in 1 ml Dimethylformamid gelöst. Man fügt bei 0°C 4 mg (0,13 mmol) Natriumhydrid und 11 µl (0,13 mmol) Ethyliodid zu, läßt 3 h bei 0°C rühren, verdünnt mit Wasser und extrahiert 3 mal mit Ether. Die organische Phase wird getrocknet ($MgSO_4$) und eingeengt. Der Rückstand wird dann an Kieselgel mit Dichlormethan/Methanol (50:1) als Eluens chromatographiert.
Ausbeute: 14%
$R_f$ = 0,18 (Dichlormethan/Methanol (20:1))
MS (EI): m/z = 407 $(M+H)^+$

**Patentansprüche**

**1.** Verwendung von substituierten Chromanen der allgemeinen Formel (I)

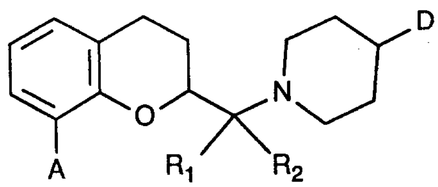

in welcher
A für Wasserstoff, Hydroxy oder geradkettiges oder verzweigtes Alkoxy mit bis zu 8

Kohlenstoffatomen steht,

R[1] und R[2] für Wasserstoff stehen,

oder

R[1] und R[2] gemeinsam für die -C = O-Gruppe stehen,

D für einen Rest der Formel $-NR^3-CO-NR^4R^5$ oder

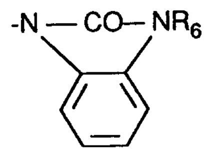

steht,

worin

R[3], R[4] und R[6] gleich oder verschieden sind und Wasserstoff oder geradkettiges oder verzweigtes Alkyl mit bis zu 6 Kohlenstoffatomen bedeuten,

R[5] Aryl mit 6 bis 10 Kohlenstoffatomen bedeutet, das gegebenenfalls bis zu 3-fach gleich oder verschieden durch Halogen, Hydroxy, Carboxy, Trifluormethyl, Trifluormethoxy, durch geradkettiges oder verzweigtes Acyl, Alkoxy oder Alkoxycarbonyl mit jeweils bis zu 8 Kohlenstoffatomen oder durch geradkettiges oder verzweigtes Alkyl oder Alkenyl mit jeweils bis zu 8 Kohlenstoffatomen substituiert ist, die ihrerseits durch Hydroxy, Carboxy oder durch geradkettiges oder verzweigtes Alkoxy oder Alkoxycarbonyl mit jeweils bis zu 6 Kohlenstoffatomen substituiert sein können

und deren Racemate, Enantiomere und Salze,

zur Herstellung von Arzneimitteln zur Bekämpfung von HIV.

**2.** Verwendung von Verbindungen der allgemeinen Formel (I) gemäß Anspruch 1, in welcher

A für Wasserstoff, Hydroxy oder geradkettiges oder verzweigtes Alkoxy mit bis zu 6 Kohlenstoffatomen steht,

R[1] und R[2] für Wasserstoff stehen,

oder

R[1] und R[2] gemeinsam für die -C = O-Gruppe stehen,

D für einen Rest der Formel $-NR^3-CO-NR^4R^5$ oder

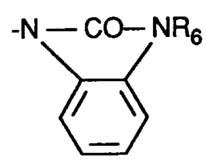

steht,

worin

R[3], R[4] und R[6] gleich oder verschieden sind und Wasserstoff oder geradkettiges oder verzweigtes Alkyl mit bis zu 4 Kohlenstoffatomen bedeuten,

R[5] Phenyl oder Naphthyl bedeutet, die gegebenenfalls bis zu 2-fach gleich oder verschieden durch Fluor, Chlor, Brom, Hydroxy, Carboxy, Trifluormethyl, Trifluormethoxy, durch geradkettiges oder verzweigtes Acyl, Alkoxy oder Alkoxycarbonyl mit jeweils bis zu 6 Kohlenstoffatomen, oder durch geradkettiges oder verzweigtes Alkyl oder Alkenyl mit jeweils bis zu 6 Kohlenstoffatomen substituiert sind, die ihrerseits durch Hydroxy, Carboxy oder durch geradkettiges oder verzweigtes Alkoxy oder Alkoxycarbonyl mit jeweils bis zu 4 Kohlenstoffatomen substituiert sein können,

und deren Racemate, Enantiomere und Salze,

zur Herstellung von Arzneimitteln zur Bekämpfung von HIV.

**3.** Verwendung von Verbindungen der allgemeinen Formel (I) gemäß Anspruch 1, in welcher

A für Wasserstoff, Hydroxy oder geradkettiges oder verzweigtes Alkoxy mit bis zu 5 Kohlenstoffatomen steht,

$R^1$ und $R^2$ für Wasserstoff stehen,

oder

$R^1$ und $R^2$ gemeinsam für die -C = O-Gruppe stehen,

D für einen Rest der Formel $-NR^3-CO-NR^4R^5$ oder

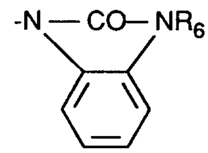

steht,

worin

$R^3$, $R^4$ und $R^6$ gleich oder verschieden sind und Wasserstoff oder geradkettiges oder verzweigtes Alkyl mit bis zu 3 Kohlenstoffatomen bedeuten,

$R^5$ Phenyl oder Naphthyl bedeutet, die gegebenenfalls bis zu 2-fach gleich oder verschieden durch Fluor, Chlor, Brom, Trifluormethyl, Trifluormethoxy, durch geradkettiges oder verzweigtes Acyl, Alkoxy oder Alkoxycarbonyl mit jeweils bis zu 5 Kohlenstoffatomen, oder durch geradkettiges oder verzweigtes Alkyl oder Alkenyl mit jeweils bis zu 5 Kohlenstoffatomen substituiert sind, die ihrerseits durch Hydroxy, Carboxy oder durch geradkettiges oder verzweigtes Alkoxy oder Alkoxycarbonyl mit jeweils bis zu 3 Kohlenstoffatomen substituiert sein können,

und deren Racemate, Enantiomere und Salze,

zur Herstellung von Arzneimitteln zur Bekämpfung von HIV.

**4.** Verbindungen der allgemeinen Formel (Ia),

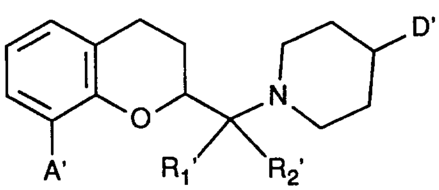

**(Ia)**

in welcher

A' für Wasserstoff, Hydroxy oder geradkettiges oder verzweigtes Alkoxy mit bis zu 8 Kohlenstoffatomen bedeutet,

$R^{1'}$ und $R^{2'}$ für Wasserstoff stehen,

oder

$R^{1'}$ und $R^{2'}$ gemeinsam für die -C = O-Gruppe stehen,

D für einen Rest der Formel $-NR^{3'}-CO-NR^{4'}R^{5'}$ oder

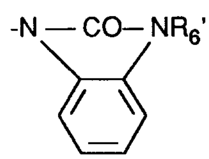

steht,

worin

$R^{3'}$, $R^{4'}$ und $R^{6'}$ gleich oder verschieden sind und Wasserstoff oder geradkettiges oder verzweigtes Alkyl mit bis zu 6 Kohlenstoffatomen bedeuten,

$R^{5'}$ Aryl mit 6 bis 10 Kohlenstoffatomen bedeutet, das gegebenenfalls bis zu 3-fach gleich oder verschieden durch Halogen, Hydroxy, Carboxy, Trifluormethyl, Trifluormethoxy, durch geradkettiges oder verzweigtes Acyl, Alkoxy oder Alkoxycarbonyl mit jeweils bis zu 8 Kohlenstoffatomen oder durch geradkettiges oder verzweigtes Alkyl oder Alkenyl mit jeweils bis zu 8 Kohlenstoffatomen substituiert ist, die ihrerseits durch Hydroxy, Carboxy oder durch geradkettiges oder verzweigtes Alkoxy oder Alkoxycarbonyl mit jeweils bis zu 6 Kohlenstoffatomen substituiert sein können

und deren Racemate, Enantiomere und Salze,
mit der Maßgabe, daß D' nicht für den Rest der Formel

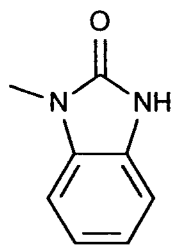

stehen darf, wenn im Fall des Racemats und der Enantiomere A', $R^{1'}$ und $R^{2'}$ Wasserstoff bedeuten, oder im Fall des Racemats, A' für Methoxy steht und $R^1$ und $R^2$ Wasserstoff bedeuten.

**5.** Verfahren zur Herstellung der Verbindungen der allgemeinen Formeln (I) und (Ia), dadurch gekennzeichnet, daß man
im Fall, daß D und D' jeweils entsprechend für die Reste der Formeln $-NR^3-CO-NR^4R^5$ oder $-NR^{3'}-CO-NR^{4'}R^5$ stehen,

[A] Verbindungen der allgemeinen Formel (II)

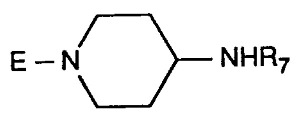 (II)

in welcher

$R^7$ die in den Ansprüchen 1 bis 4 angegebene Bedeutung von $R^3$ und $R^{3'}$ umfaßt

und

E für eine Aminoschutzgruppe, vorzugsweise für Benzyl steht,

zunächst durch Umsetzung mit Isocyanaten der allgemeinen Formel (III)

$O=C=N-R^8$ (III)

in welcher

$R^8$ die in den Ansprüchen 1 bis 4 angegebene Bedeutung von $R^5$ und $R^{5'}$ umfaßt,

in inerten Lösemitteln, gegebenenfalls in Anwesenheit einer Base und/oder eines Hilfsmittels, in die Verbindungen der allgemeinen Formel (IV)

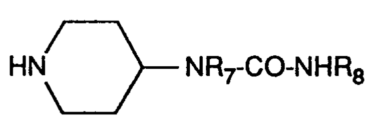 (IV)

in welcher

$R^7$ und $R^8$ die in den Ansprüchen 1 bis 4 angegebene Bedeutung haben,

überführt,

und anschließend mit Verbindungen der allgemeinen Formel (V)

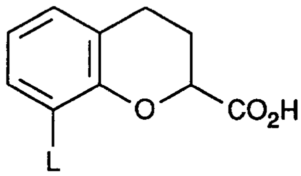

(V)

in welcher

L die in den Ansprüchen 1 bis 4 angegebene Bedeutung von A und A' umfaßt, aber nicht für Hydroxy steht,

ebenfalls in inerten Lösemitteln, gegebenenfalls in Anwesenheit einer Base und eines Hilfsstoffes umsetzt,

oder

[B] im Fall, daß D und D' jeweils entsprechend für die Reste der Formeln

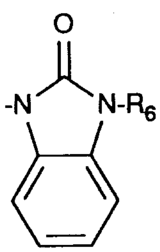

oder

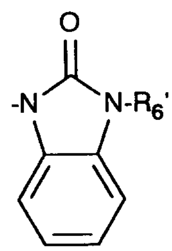

stehen,

Verbindungen der allgemeinen Formel (V) mit Verbindungen der allgemeinen Formel (VI)

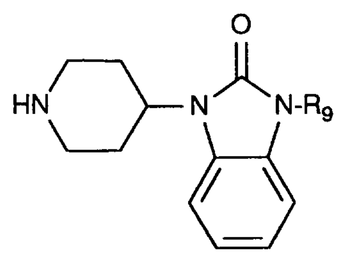

(VI)

in welcher

$R^9$ die in den Ansprüchen 1 bis 4 angegebene Bedeutung von $R^6$ und $R^{6'}$ umfaßt,

in inerten Lösemitteln, in Anwesenheit einer Base und/oder Hilfsmittels umsetzt,

und im Fall, $R^1/R^{1'}, R^2$ = H die Carbonylfunktion nach üblichen Methoden reduziert,

und im Fall L = OH die entsprechende Methoxyverbindung mit Säuren spaltet,

und im Fall L = Alkoxy die entsprechenden Hydroxyverbindungen verethert,

und im Fall $R^7$, $R^4/R^{4'}$ und/oder $R^9 \neq$ H gegebenenfalls eine Alkylierung durchführt.

Europäisches Patentamt

# EUROPÄISCHER RECHERCHENBERICHT

Nummer der Anmeldung
EP 94 10 8126

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X,P | EP-A-0 546 388 (BAYER A.G.) 16. Juni 1993<br>* Ansprüche 1-10 *<br>----- | 4,5 | A61K31/44<br>C07D405/06<br>C07D405/14 |

| RECHERCHIERTE SACHGEBIETE (Int.Cl.5) |
|---|
| A61K<br>C07D |

Der vorliegende Recherchenbericht wurde für alle Patentansprüche erstellt

| Recherchenort | Abschlußdatum der Recherche | Prüfer |
|---|---|---|
| DEN HAAG | 8. August 1994 | Theuns, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P04C03)